# EUROPEAN PATENT APPLICATION

(11) **EP 1 579 771 A1**
(43) Date of publication of application: **28.09.2005**
(21) Application number: 03778745.4
(22) Date of filing: 26.11.2003
(51) Int. Cl.: A23L 1/00, A61K 9/34, A23G 3/00, A23G 1/00

(54) **WATER-BASED SHELLAC COATING MATERIAL, PROCESS FOR PRODUCING THE SAME, COATED FOOD OBTAINED WITH THE COATING MATERIAL, PROCESS FOR PRODUCING THE SAME, COATED MEDICINE, PROCESS FOR PRODUCING THE SAME, GLAZING COMPOSITION FOR OILY SNACK, METHOD OF GLAZING, AND GLAZED OILY SNACK**

(30) Priority: 29.11.2002 JP 2002347465; 20.06.2003 JP 2003176333; 13.08.2003 JP 2003293002; 10.09.2003 JP 2003318500
(71) Applicant: Freund Corporation, Tokyo 163-6034 (JP)
(72) Inventor: SHOBU, Tomoyuki, c/o Freund Corporation, Hamamatsu-shi, Shizuoka 431-2103 (JP); IGUSA, Kazuo, c/o Freund Corporation, Hamamatsu-shi, Shizuoka 431-2103 (JP); OGASAWARA, Toshichika, c/o Freund Corporation, Hamamatsu-shi, Shizuoka 431-2103 (JP); MOCHIZUKI, Keizou, Saitama 350-0225 (JP); YAMADA, Kazumi, c/o Freund Chemical Co.,Ltd., Saitama 366-0027 (JP); HARA, Hidejiro, c/o Freund Corporation, Shinjuku-ku, Tokyo 163-6034 (JP)
(74) Representative: Eisenführ, Speiser & Partner
(86) International application number: PCT/JP2003/015097
(87) International publication number: WO 2004/049820

(57) **Abstract**

There are provided an aqueous shellac coating agent comprising shellac, a basic amino acid and/or a basic phosphate, as well as a production process therefor; a coated food and a coated drug that have been coated with such a coating agent; a glazing composition for oil-based confectionary which is in a liquid form and comprises an aqueous shellac solution (A) containing shellac, a basic amino acid and/or a basic phosphate dissolved in water, a thickener (B), and/or a sugar (C); a process for glazing oil-based confectionary in which this glazing composition is applied to oil-based confectionary to be glazed, thereby generating a glaze; and glazed oil-based confectionary produced using this process for glazing oil-based confectionary.

## Description

### Technical Field

The present invention relates to an aqueous shellac coating agent with excellent enteric properties, acid resistance, masking characteristics, moisture resistance, gloss, and stability, as well as a process for producing such a shellac coating agent; a coated food and a coated drug covered with such a shellac coating agent, and processes for producing such items; together with a glazing composition used for glazing oil-based confectionary containing chocolate, white chocolate or nut cream or the like, a process for glazing oil-based confectionary, and glazed oil-based confectionary produced by such a process.

### Background Art

Shellac is produced mainly in India, Thailand and the south of China, and is a resin type material obtained from the secretions of Laccifer Lacca insects that live as parasites on shrubs such as beans and mulberries. Shellac is a natural product comprising resin acid esters of aleuritic acid and shellolic acid, or aleuritic acid and jalaric acid as a primary component. Shellac is recorded within Japan's Specifications and Standards for Food Additives, as well as in the Japanese Pharmacopoeia, the United States Pharmacopoeia, and the European Pharmacopoeia. It is recorded under the name "shellac" in Japan's Specifications and Standards for Food Additives, whereas in the Japanese Pharmacopoeia, the product obtained by refining the crude product is recorded under the name "refined shellac", and the product obtained by subsequent bleaching is listed under the name "white shellac". Because shellac has film forming properties, it provides an ideal edible coating, derived from a natural product and offering high levels of safety, and is widely used as a coating for confectionery, medication tablets, seeds, and fruit and the like, and as a raw material in paints and inks. The coloring of the shellac coating differs depending on the degree of refining. A coating formed from a typical refined shellac is a dark brown color, whereas coatings formed from white shellac or decolorized shellac that have undergone additional decolorization treatment can be light yellow, or even very faintly yellow, and consequently the color can be selected depending on the intended purpose or application. In the case of foodstuffs or drugs, the external appearance is often extremely important, and so decolorized shellac or white shellac is preferably used as the coating agent. In most cases, the shellac coating is used in the form of a solution produced by dissolving the shellac in a solvent such as an alcohol like ethanol.

Examples of the methods used for coating the shellac onto a foodstuff or a drug include methods in which the target objects to be coated, such as tablets, are immersed in an alcohol solution of shellac, and subsequently dried, thereby forming a coating on the surface of the target objects, and methods in which a shellac solution is sprayed onto the target objects to be coated using either cold air or hot air aeration, thereby forming a coating. A coating formed by one of these methods displays enteric properties, acid resistance, gloss, and moisture resistance, and can be used for:
- preventing the deactivation of acid intolerant enzymes and lactic acid bacteria in gastric acid, and imparting enteric properties,
- masking the taste of bitter materials such as vitamins, and
- preventing moisture absorption by sugars, and moisture proofing deliquescent materials.

However, when an alcohol solution of shellac is used in the coating process, a problem arises in that stringiness can develop as a result of increased stickiness during coating. In the case where the shellac has been coated onto tablets for example, this stringiness can lead to partial separation within the coating film, leading to a vastly inferior external appearance for the coated tablets, and an increased likelihood of rejects. In addition, because large quantities of organic solvent are used in the production methods described above, an additional problem arises in terms of the accumulated costs associated with installing fire extinguishing equipment and the like at the production facility, and initiating measures to ensure the health and safety of staff, and prevent environmental pollution.

Furthermore, another characteristic of shellac coatings is that they tend to degenerate over time, and consequently in those cases where a shellac coating agent is used as an enteric coating material, this enteric property is gradually lost over time, meaning the coating becomes insoluble in the intestine, which represents a major drawback.

Conventionally, in order to overcome the problems associated with shellac described above, the following types of measures have been proposed. (1) It has been proposed that the problem of stringiness occurring during coating can be prevented by combining the shellac with a vegetable oil, an animal oil or a wax or the like (for example, see patent reference 1). (2) Methods that avoid the use of organic solvents in the shellac solution by forming an aqueous solution using an alkali metal hydroxide such as sodium hydroxide or ammonia are well known, and a method for obtaining an oil resistant coating from an aqueous shellac solution produced using ammonia water has been proposed (for example, see patent reference 2). (3) A method of suppressing the degeneration of the coating over time by combining the shellac with tocopherol has also been proposed (for example, see patent reference 3).

However, in the methods (1) and (3) described above, the existing problems associated with organic solvent use remain. Furthermore in the method (2), if ammonia water is used, then the produced coating has a significant drawback in that it is very prone to color change and degeneration over time. Furthermore, if an aqueous shellac solution produced using sodium hydroxide is used for coating tablets, then even if a shellac that has undergone decolorization treatment is used, the produced coating is either brown or a red-brown color, leading to a potential decrease in the commercial value of coated foodstuffs or drugs. Furthermore during coating, the reduction in workability associated with stringiness is a considerable problem, and this stringiness is particularly marked when white shellac is used. Preventing such problems from arising places a considerable workload on producers.

In addition, in terms of the enteric properties of coated tablets, it is difficult to achieve a coating that displays resistance to gastric juices and yet disintegrates in intestinal juices using either the method (1) or the method (3) above, whereas in the method (2), if for example a shellac solution is produced using sodium hydroxide, then penetration by gastric juices while the tablet is still in the stomach can cause considerable swelling of the tablet, inviting leakage of the tablet contents, and in extreme cases the tablet may actually disintegrate while still in the stomach, meaning the desired enteric function is not accomplished.

As described above, a large number of techniques have been investigated as potential solutions to the problems associated with shellac coating agents, but even by combining these different techniques, it has not been possible to resolve the existing problems without generating new problems, and consequently a resolution of the above problems has been keenly sought.

On the other hand, conventional processes for glazing the surface of oil-based confectionary containing chocolate, white chocolate or nut cream or the like in order to impart gloss to the product have typically utilized shellac ethanol solutions. However, when an ethanol solution of shellac is coated directly onto a chocolate product using a conventional glazing process, the solution affects the chocolate or the product being coated, meaning the desired level of gloss cannot be obtained. In order to overcome this problem, a glazing process has been proposed in which an undercoat solution, comprising a sugar solution of sugar or starch syrup to which has been added gum arabic, dextrin, and a colloid of starch sugar, is first applied to the product to produce the desired gloss, and an alcohol solution of shellac is subsequently applied to ensure a more enduring gloss (for example, see the non-patent reference 1).

In this conventional process, the reason that the alcohol solution of shellac is applied after the undercoat liquid has been used to generate the desired gloss, is that the undercoat solution alone does not provide sufficient durability for the glaze, and the alcohol solution of shellac is required to ensure the preservation of the glaze.

Furthermore, another process for glazing food has been disclosed in which instead of shellac, the foodstuff is coated with a mixed solution produced by adding a liquid fatty acid and/or a polyglycerol fatty acid ester to a solution containing corn protein zein dissolved in ethanol and/or isopropanol (see patent reference 4).

However; as both of the conventional glazing processes disclosed in the aforementioned non-patent reference 1 and the patent reference 4 utilize a volatile organic solvent such as ethanol or isopropanol, strict fire prevention measures must be put in place at production sites, and not only does the additional fire extinguishing equipment and solvent removal equipment increase the size of the production facility and contribute to increased costs, but the transpiration of organic solvent vapor such as alcohol or the like generated during the glazing process is also undesirable, both in terms of its deleterious effect on the workplace environment, and in terms of the associated atmospheric and environmental pollution it generates.

In contrast, glazing processes that utilize hemicellulose derived from soybeans, whey protein or a lactoprotein as the glazing agent, and do not require the use of volatile organic solvents, have also been proposed, but a glazing process that is able to provide the same level of gloss as that obtained by a shellac glazing process, while also offering good durability of that gloss has yet to be developed.
(Patent Reference 1)
   Japanese Unexamined Patent Application, First Publication No. Hei 8-311405
(Patent Reference 2)
   Japanese Unexamined Patent Application, First Publication No. 2002-1864
(Patent Reference 3)
   Japanese Unexamined Patent Application, First Publication No. Sho 55-162715
(Patent Reference 4)
   Japanese Unexamined Patent Application, First Publication No. Hei 10-108630
(Non-Patent Reference 1)
   Industrial Chocolate Manufacture and Use - Third Edition: pp 297 to 298

### Disclosure of the Invention

The present invention takes the above circumstances into consideration, with an object of providing an aqueous shellac coating agent with excellent enteric properties, acid resistance, masking characteristics, moisture resistance, gloss, and stability, as well as a process for producing such a shellac coating agent, and a coated food and a coated drug covered with such a shellac coating agent.

Furthermore, another object of the present invention is to provide a glazing composition containing an aqueous shellac coating agent for imparting an attractive glaze to the surface of oil-based confectionary such as spherical chocolates or the like without requiring the use of an organic solvent, together with a process for glazing oil-based confectionary that uses such a glazing composition, and oil-based confectionary that has been glazed by such a process.

In order to achieve the above objects, the present invention provides an aqueous shellac coating agent comprising shellac, a basic amino acid, and/or a basic phosphate. Furthermore, the present invention also provides an aqueous shellac coating agent in which a basic amino acid and/or a basic phosphate is contained in shellac.

In an aqueous shellac coating agent according to the present invention, the basic amino acid described above is preferably one or more materials selected from a group consisting of arginine, lysine, and ornithine.

The aforementioned basic phosphate is preferably one or more materials selected from a group consisting of trisodium phosphate, tripotassium phosphate, disodium hydrogenphosphate, dipotassium hydrogenphosphate, tetrasodium pyrophosphate, and tetrapotassium pyrophosphate.

In an aqueous shellac coating agent of the present invention, the quantity of the basic amino acid is preferably within a range from 0.05 to 0.40 parts by weight per 1 part by weight of shellac.

The quantity of the basic phosphate is preferably within a range from 0.04 to 0.60 parts by weight per 1 part by weight of shellac.

An aqueous shellac coating agent of the present invention may also contain one or more materials selected from a group consisting of aliphatic polyols, fatty acid esters, water soluble sugars, triethyl citrate, polyethylene glycol, and sodium lactate.

The aliphatic polyol described above is preferably one or more compounds selected from a group consisting of glycerol, propylene glycol, and sugar alcohols. The sugar alcohol is one or more compounds selected from a group consisting of sorbitol, maltitol, erythritol, xylitol, mannitol, palatinit, and lactitol.

The aforementioned fatty acid ester is preferably one or more compounds selected from a group consisting of sucrose fatty acid esters, mono-, di-, tri- or polyglycerol fatty acid esters, organic acid monoglycerides, propylene glycol fatty acid esters, sorbitan fatty acid esters, and polysorbates.

The aforementioned water soluble sugar is preferably one or more compounds selected from a group consisting of trehalose, oligosaccharides, maltose, galactose, lactose, sucrose, glucose, and fructose.

Furthermore, the present invention also provides a process for producing an aqueous shellac coating agent, comprising the steps of mixing the shellac with a basic amino acid solution, a basic phosphate solution, or a mixed solution of a basic amino acid and a basic phosphate, preparing an aqueous shellac coating liquid with the shellac stably dissolved or dispersed therein, and where necessary, concentrating or drying the coating liquid.

In addition, the present invention also provides a process for producing an aqueous shellac coating agent, comprising the steps of dispersing the shellac in a solution of an acidic material, subsequently adding a basic alkali metal salt to the solution, preparing an aqueous shellac coating liquid with the shellac stably dissolved or dispersed therein, and where necessary, concentrating or drying the coating liquid.

In this process for producing an aqueous shellac coating agent, the basic alkali metal salt is preferably one or more compounds selected from a group consisting of alkali metal hydroxides, carbonates, and bicarbonates.

The acidic material is preferably one or more compounds selected from a group consisting of phosphoric acid and polyphosphoric acid.

A process for producing an aqueous shellac coating according to the present invention preferably comprises an inert gas treatment step for passing inert gas through the aqueous shellac coating liquid and replacing any gas within the liquid.

The inert gas is preferably one or more gases selected from a group consisting of nitrogen, argon, and helium.

Furthermore, the present invention also provides a coated food comprising a food coated with an aforementioned aqueous shellac coating agent.

In addition, the present invention also provides a coated food with a multi-layered coating, comprising a layer containing an aforementioned aqueous shellac coating agent as a primary component, and a layer containing another coating agent as a primary component.

The other coating agent described above is preferably formed from one or more materials selected from a group consisting of hydroxypropylmethylcellulose, methylcellulose, ethylcellulose, shellac, zein, components derived from yeast cellular walls, water soluble polysaccharides, fats, oils, waxes, and chitosan.

Furthermore, the present invention also provides a process for producing a coated food comprising a step for coating the food with a coating liquid containing 1 to 50% by weight of an aqueous shellac coating agent, thereby forming the coated food, wherein the shellac solid fraction content within the produced coated food is within a range from 0.1 to 50% by weight.

Furthermore, the present invention also provides a coated drug comprising a drug coated with an aforementioned aqueous shellac coating agent.

In addition, the present invention also provides a coated drug comprising a drug covered with a coating containing an aforementioned aqueous shellac coating agent and a drug component.

Furthermore, the present invention also provides a coated drug with a multi-layered coating, comprising a layer containing an aforementioned aqueous shellac coating agent as a primary component, and a layer containing another coating agent as a primary component.

In addition, the present invention also provides a coated drug with a multi-layered coating, comprising a layer containing an aforementioned aqueous shellac coating agent and a drug component, and a layer containing another coating agent as a primary component.

The other coating agent described above is preferably formed from one or more materials selected from a group consisting of methacrylic acid copolymers, hydroxypropylmethylcellulose, hydroxypropylmethylcellulose phthalate, methylcellulose, ethylcellulose, shellac, zein, components derived from yeast cellular walls, water soluble polysaccharides, fats, oils, waxes, and chitosan.

Furthermore, the present invention also provides a process for producing a coated drug comprising a step for coating the drug with a coating liquid containing 1 to 50% by weight of an aqueous shellac coating agent, thereby forming the coated drug, wherein the shellac solid fraction content within the produced coated drug is within a range from 0.1 to 50% by weight.

In addition, the present invention also provides a glazing composition for oil-based confectionary, which is in a liquid form and comprises an aqueous shellac solution (A) produced by dissolving an aqueous shellac coating agent formed from a mixture of shellac, a basic amino acid and/or a basic phosphate in water, a thickener (B), and/or a sugar (C). The aqueous shellac solution (A) is an aqueous solution containing an aforementioned aqueous shellac coating agent of the present invention, comprising shellac, a basic amino acid and/or a basic phosphate.

In a glazing composition of the present invention, the basic amino acid added to the aqueous shellac solution (A) is preferably one or more materials selected from a group consisting of arginine, lysine, and ornithine.

The basic phosphate added to the aqueous shellac solution (A) is preferably one or more materials selected from a group consisting of trisodium phosphate, tripotassium phosphate, disodium hydrogenphosphate, dipotassium hydrogenphosphate, tetrasodium pyrophosphate, and tetrapotassium pyrophosphate.

The quantity of the basic amino acid added to the aqueous shellac solution (A) is preferably within a range from 0.05 to 0.40 parts by weight per 1 part by weight of shellac.

The quantity of the basic phosphate added to the aqueous shellac solution (A) is preferably within a range from 0.04 to 0.60 parts by weight per 1 part by weight of shellac.

The aqueous shellac coating agent preferably accounts for 1 to 40% by weight of the aqueous shellac solution (A).

The aforementioned thickener (B) is preferably one, or a mixture of two or more materials selected from a group consisting of pullulan, xanthan gum, guar gum, locust bean gum, tamarind gum, pectin, carrageenan, tragacanth gum, gum arabic, gelatin, and collagen.

The aforementioned sugar (C) is preferably one, or a mixture of two or more materials selected from a group consisting of monosaccharides, disaccharides, oligosaccharides, acid-saccharified starch syrup, enzyme-saccharified starch syrup, and starch decomposition products.

The glazing composition for oil-based confectionary may also contain a sugar alcohol instead of the sugar (C).

The sugar alcohol is preferably one, or a mixture of two or more materials selected from a group consisting of reduced starch syrup, sorbitol, maltitol, and xylitol.

The concentration of the sugar (C) is preferably within a range from 10 to 80% by weight.

The aforementioned glazing composition preferably contains essentially no organic solvents.

Furthermore, the present invention also provides a process for glazing oil-based confectionary, in which an aforementioned glazing composition is applied to oil-based confectionary to be glazed to generate a glaze.

The process for glazing oil-based confectionary according to the present invention preferably comprises the steps of applying a glazing composition to the oil-based confectionary, and polishing.

Furthermore, in such a process of the present invention, the glazing composition is preferably added and applied while the oil-based confectionary is rolled within a rotary pan, and the glazed confectionary is preferably subsequently subjected to forced-air drying.

In such a process of the present invention, the oil-based confectionary is preferably one or more types of confectionary selected from a group consisting of chocolate, white chocolate and nut cream.

The process of the present invention preferably uses essentially no organic solvents.

The present invention also provides glazed oil-based confectionary produced using the above process for glazing oil-based confectionary. Furthermore, the present invention also provides glazed oil-based confectionary that has undergone glazing treatment using the process for glazing oil-based confectionary.

Glazed oil-based confectionary of the present invention is preferably granular oil-based confectionary comprising one or more types of confectionary selected from a group consisting of chocolate, white chocolate and nut cream.

In addition, confectionary of the present invention is preferably glazed oil-based confectionary obtainable by using a process for glazing oil-based confectionary described above to glaze granular oil-based confectionary that has been produced by coating edible granules of a material selected from a group consisting of chocolate, oil-based cream, nuts, and candy with a material selected from a group consisting of oil-based cream, chocolate and white chocolate, and performing subsequent molding.

According to the present invention, an aqueous shellac coating agent with excellent handling properties, quality, and stability can be provided, together with a coated food and a coated drug that have been covered with such a shellac coating agent.

Furthermore, the present invention also enables an attractive glaze to be imparted to the surface of oil-based confectionary, without requiring the use of organic solvents.

In addition, because the present invention enables an attractive glaze to be imparted to the surface of oil-based confectionary without requiring the use of organic solvents, safety during production can be improved, and any deleterious impact on the environment can be prevented.

### Best Mode for Carrying out the Invention

As follows is a detailed description of embodiments of the present invention.

As a result of intensive investigations aimed at achieving the objects described above, the inventors of the present invention discovered that by adding a basic amino acid, and/or a basic phosphate to shellac, an aqueous shellac coating agent could be obtained that resolved the problems associated with the conventional technology described above, and were hence able to complete the present invention.

In other words, the present invention relates to an aqueous coating agent formed from a composition produced by dissolving, or partially dissolving, shellac, which is insoluble in water under neutral conditions or acidic conditions, in water in the presence of a basic amino acid and/or a basic phosphate, as well as foodstuffs and drugs coated with such a coating agent.

In this document, the term "aqueous" means that the shellac coating agent is either dissolved or dispersed in water, that is, the shellac coating agent is either water soluble or water dispersible.

The formation of an aqueous coating agent refers to the acquisition, by a shellac that is insoluble in water under neutral conditions or acidic conditions, such as purified shellac, decolorized shellac or white shellac, of the "aqueous" property described above, through the addition of a basic amino acid such as arginine and/or a basic phosphate such as trisodium phosphate to the shellac.

The term "basic phosphate" refers to a phosphate salt that forms an aqueous solution that displays basicity.

The term "coating agent" is not restricted to the coating agents used in fields such as the production of foodstuffs or the production of drugs, but refers to any coating agent (also referred to by other names such as film forming agent) that is used in any of a variety of fields to form a coating on an object or product.

The process of "coating" refers to the application of a coating agent of the present invention to a target object to be coated such as a food or a drug, thereby covering at least a portion of the surface of the target object with the coating agent. Furthermore, the coating need not necessarily be formed as the outermost layer on the target object, and configurations in which the coating film is over-coated, or configurations in which the coated product is encased within a capsule are also possible.

The term "food" refers to all foodstuffs that are edible by people or animals.

In the present invention, in addition to typical foodstuffs such as confectionary, the term "food" also includes coated health food products produced by covering health foods in a coating that has gastric acid resistance and intestinal juice disintegration properties. Specifically, for health foods in which it is desirable that the components such as lactic acid bacteria, nattokinase, royal jelly, lactoferrin do not lose their activity in gastric acid, but are rather absorbed within the intestine, a coating agent of the present invention is ideal for imparting the required enteric properties.

The term "drug" refers to all types of drugs that can be administered to people or animals.

Examples of typical digestive system organ drugs include benzimidazole based medications with antiulcer properties such as 2-{[3-methyl-4-(2,2,2-trifluoroethoxy)-2-pyridyl]methylsulfinyl]benzimidazole and 5-methoxy-2-[(4-methoxy-3,5-dimethyl-2-pyridyl)methylsulfinyl]benzimidazole, as well as cimetidine, ranitidine, pancreatin, bisacodyl, and 5-aminosalicylic acid.

Examples of typical central nervous system drugs include aspirin, indomethacin, diazepam, idebenone, ibuprofen, paracetamol, naproxen, piroxicam, diclofenac, sulindac, lorazepam, nitrazepam, phenytoin, acetaminophen, ethenzamide, and ketoprofen.

Examples of typical circulatory system drugs include molsidomine, vinpocetine, propranolol, methyldopa, dipyridamole, furosemide, triamterene, nifedipine, atenolol, spironolactone, metoprolol, pindolol, captopril, and isosorbide nitrate.

Examples of typical respiratory system drugs include theophylline, amlexanox, dextromethorphan, pseudoephedrine, salbutamol, and guaifenesin.

Examples of typical antibiotics and chemotherapy agents include cefalexin, cefaclor, cefradine, amoxicillin, pivampicillin, bacampicillin, dicloxacillin, erythromycin, erythromycin stearate, lincomycin, doxycycline, and trimethoprim-sulfamethoxazole.

Examples of typical metabolic system drugs include serrapeptase, lysozyme chloride, adenosine triphosphate, glibenclamide, and potassium chloride.

Examples of suitable vitamin drugs include vitamin B1, vitamin B2, vitamin B6, vitamin C, and fursultiamine.

These are all drugs that contain medication that is either easily deactivated by gastric acid, or has side effects on the stomach, and preferably undergoes disintegration and absorption within the intestine, and as such they are ideally suited to coating with a coating agent of the present invention, which imparts effective enteric properties. The above list of drugs is not a restrictive list, and the present invention can be applied to any drug containing a medication that requires enteric properties.

The shellac used in the present invention can be appropriately selected from any of the various known shellacs, and may utilize materials marketed under names such as refined shellac, decolorized shellac, or white shellac. If the coloring of the coating is taken into consideration, then decolorized shellac and white shellac are preferred.

In the present invention, an aqueous coating agent is achieved by adding a basic amino acid and/or a basic phosphate to the shellac. There is no necessity for the shellac to dissolve completely, and provided any undissolved shellac exists as fine particles, then the presence of such residual undissolved shellac does not greatly impede the formation of a uniform coating. An aqueous shellac coating agent of the present invention is a coating agent in which a basic amino acid and/or a basic phosphate is contained in shellac. Furthermore, an aqueous shellac coating agent of the present invention is also a coating agent comprising shellac, a basic amino acid, and/or a basic phosphate.

The basic amino acid added can utilize any known basic amino acid such as arginine, lysine, ornithine, hydroxylysine, and histidine, but is preferably one or more materials selected from a group consisting of arginine, lysine, and ornithine, and from the viewpoint of coating workability, arginine is the most desirable. In contrast, high molecular weight basic amino acid compounds such as polylysine are ineffective in forming an aqueous shellac coating agent, and cannot be used as the sole basic amino acid.

The basic phosphate can utilize those basic phosphates that are authorized for use within the production of foodstuffs or drugs, and one or more compounds selected from a group consisting of trisodium phosphate, tripotassium phosphate, disodium hydrogenphosphate, dipotassium hydrogenphosphate, tetrasodium pyrophosphate, and tetrapotassium pyrophosphate are preferred. Generation of an aqueous coating agent using only weakly acidic salts such as sodium dihydrogenphosphate is difficult.

In the present invention, either the basic amino acid or the basic phosphate can be added in isolation to the shellac, or a combination of the compounds can be used, depending on the intended purpose or application. Furthermore, these compounds can also be used in combination with materials other than the basic amino acid and the basic phosphate, for example, basic materials that are authorized for use within the production of foodstuffs or drugs, such as sodium hydroxide, potassium hydroxide, calcium hydroxide, sodium carbonate and potassium carbonate. However, if an attempt is made to form an aqueous coating agent from a decolorized shellac using only one of the basic materials other than the basic amino acid and the basic phosphate, such as sodium hydroxide, then the product coating is either brown or a red-brown color, which is markedly different from the coloring of the coating produced by the original decolorized shellac. Alkali soluble coating agents, such as cellulose derivatives formed from ether linkages like hydroxypropylmethylcellulose phthalate, are known, but the coloring of coatings formed from aqueous solutions of these types of materials do not vary significantly depending on the basic material used to generate the aqueous coating agent. This phenomenon, where the coloring of the coating varies considerably depending on the basic material used for formation of the aqueous coating agent is observed only for shellac.

The quantities of the basic amino acid and/or the basic phosphate used in producing an aqueous shellac coating agent vary depending on the raw material shellac used, and on the type (for example, the strength of the basicity and the like) of basic amino acid or basic phosphate added, although typically the quantity of the basic amino acid is within a range from 0.05 to 0.40 parts by weight, and preferably from 0.12 to 0.29 parts by weight, per 1 part by weight of the shellac, while the quantity of the basic phosphate is typically within a range from 0.04 to 0.60 parts by weight, and preferably from 0.08 to 0.45 parts by weight, per 1 part by weight of the shellac. If the quantities of the basic amino acid and/or the basic phosphate are less than the above ranges, then the conversion of the shellac to an aqueous coating agent is unsatisfactory, and forming a favorable coating is difficult. In contrast, if the quantities of the basic amino acid and/or the basic phosphate exceed the above ranges, then the coloring of the formed coating may darken, the water resistance and acid resistance of the coating may deteriorate, and production costs will also increase. The pH of a coating solution comprising a coating agent of the present invention is preferably at least 6.0, and even more preferably within a range from 6.5 to 8.0.

The quantity added of the basic amino acid and/or the basic phosphate used in an aqueous shellac coating agent of the present invention, per 1 part by weight of shellac, describes the same quantity as the preferred content of the basic amino acid and/or the basic phosphate within the aqueous shellac coating agent per 1 part by weight of shellac. Accordingly, as described above, in an aqueous shellac coating agent of the present invention, the basic amino acid content is preferably within a range from 0.05 to 0.40 parts by weight per 1 part by weight of the shellac. Similarly, the basic phosphate content is preferably within a range from 0.04 to 0.60 parts by weight per 1 part by weight of the shellac.

An aqueous shellac coating agent of the present invention may also comprise one or more materials selected from a group consisting of aliphatic polyols, fatty acid esters, water soluble sugars, triethyl citrate, polyethylene glycol, and sodium lactate as a cracking inhibitor.

The aliphatic polyol is preferably one or more compounds selected from a group consisting of glycerol, propylene glycol, and sugar alcohols.

The sugar alcohol is preferably one or more compounds selected from a group consisting of sorbitol, maltitol, erythritol, xylitol, mannitol, palatinit, and lactitol.

The fatty acid ester is preferably one or more compounds selected from a group consisting of sucrose fatty acid esters, mono-, di-, tri- or polyglycerol fatty acid esters, organic acid monoglycerides, propylene glycol fatty acid esters, sorbitan fatty acid esters, and polysorbates.

The water soluble sugar is preferably one or more compounds selected from a group consisting of trehalose, oligosaccharides, maltose, galactose, lactose, sucrose, glucose, and fructose.

The quantity added of the aforementioned cracking inhibitor is preferably within a range from 2 to 50 parts by weight, and even more preferably from 10 to 35 parts by weight, per 100 parts by weight of the shellac within the aqueous shellac coating agent.

If the quantity of the cracking inhibitor is less than the above range, then a satisfactory coating cracking suppression effect cannot be achieved, and if the coating is stored for an extended period in a dry environment, cracks may appear in the coating. In contrast, if the quantity of the cracking inhibitor exceeds the above range, the mechanical strength of the coating deteriorates and the coating becomes sticky, both of which are undesirable.

Furthermore, by adding a fatty acid ester with a low HLB value to the aqueous shellac coating agent, an improvement can be achieved in the masking effect, which masks unpleasant tastes arising from either the product being coated or the coating agent itself. A specific example of a preferred fatty acid ester is sucrose stearate (brand name: "DK-ester F70", manufactured by Daiichi Pharmaceutical Co., Ltd.).

Of the above materials that can be used as a cracking inhibitor, glycerol is preferred in terms of the cracking suppression effect generated, but if too much glycerol is added, there is a danger that the coating can become sticky, causing individually coated food items or drug items to adhere to one another or clump together in lumps, causing a deterioration in coating workability. Sorbitol suffers from the same drawback as glycerol in terms of the coating workability.

Fatty acid esters do display a cracking suppression effect, although that effect is not as pronounced as that of glycerol or sorbitol. However, some fatty acid esters provide additional effects, such as improving the coating workability, and improving the gastric juice resistance and the enteric properties of the aqueous shellac coating, and consequently, by adding a combination of a fatty acid ester and either glycerol or sorbitol to the aqueous shellac coating agent, the superior cracking suppression effect of glycerol or sorbitol can be obtained, while the workability and gastric juice resistance is also improved.

By adding a cracking inhibitor to an aqueous shellac coating agent of the present invention, drugs or the like that have been coated with the aqueous shellac coating agent can be sealed in a dry environment with a desiccant such as silica gel, and stored for extended periods without any concern of cracks developing in the coating. If cracks develop in the coating of a coated foodstuff or drug during storage, then the water resistance and the acid resistance of the coating will deteriorate, and the enteric properties may also be deleteriously affected, and so by adding an aforementioned cracking inhibitor to coatings of the present invention, the danger of such cracking is removed, and the water resistance and acid resistance of the coating can be maintained at favorable levels. This cracking suppression effect of the coating is particularly important for coatings used with enteric coated foods or drugs.

An aqueous shellac coating agent of the present invention is preferably subjected to a final treatment with an inert gas. Specific examples of the inert gas include nitrogen, argon, and helium, and one or more of these inert gases is preferably bubbled through the coating agent. Treatment of the aqueous shellac coating agent with an inert gas enables the removal of components such as oxygen, which can impair the quality and stability of the coating agent, and is a preferred treatment. The residual dissolved oxygen concentration within the aqueous shellac coating agent is preferably reduced to no more than 2 mg/L.

An aqueous shellac coating agent of the present invention can be produced by a variety of processes, including a process in which the shellac is dispersed in water, and a basic amino acid and/or a basic phosphate is then added, or a process in which the shellac is added to an aqueous solution containing a basic amino acid and/or a basic phosphate dissolved in water.

In those cases where an aqueous shellac coating agent is produced using a basic amino acid, it is preferable that a solution containing the basic amino acid such as arginine dissolved in water is first prepared, and the shellac is then added to this basic amino acid solution and stirred to form an aqueous shellac coating liquid with the shellac stably dissolved or dispersed therein. This aqueous shellac coating liquid may be either used as is, or if necessary may be concentrated or dried. In addition, the aqueous shellac coating agent may also be diluted with, or dissolved in, water or another solution in order to adjust the concentration.

In another preferred production process, the shellac is dispersed in a solution of an acidic material, and a basic alkali metal salt is then added to the solution to form an aqueous shellac coating liquid with the shellac stably dissolved or dispersed therein. This aqueous shellac coating liquid may be either used as is, or if necessary may be concentrated or dried. In addition, the aqueous shellac coating agent may also be diluted with, or dissolved in, water or another solution in order to adjust the concentration. In this production process, the basic alkali metal salt is preferably one or more compounds selected from a group consisting of alkali metal hydroxides, carbonates, and bicarbonates. The acidic material can utilize organic acids, hydrochloric acid, sulfuric acid, nitric acid, phosphoric acid, or polyphosphoric acid or the like, although one or more compounds selected from a group consisting of phosphoric acid and polyphosphoric acid are preferred.

An aqueous shellac coating agent of the present invention can be used for coating food or drug formulations such as tablets, granules or capsules, and enables the production of a coated food or a coated drug according to the present invention, which displays functions such as enteric properties, acid resistance, masking characteristics, moisture resistance, gloss, and stability. Furthermore, in the case of capsules, the coating agent of the present invention may also be added in advance to the encapsulating base material.

Accordingly, a coated food in which a foodstuff has been coated with an aqueous shellac coating agent of the present invention, and a coated drug in which a drug has been similarly coated, can be obtained.

Specific examples of actual uses of the coating include adding gloss to sugar-coated tablets or chocolate; masking the taste of vitamin tablets (particularly vitamin B1), health food products such as ginkgo biloba extract, and other very bitter medications such as berberine chloride and quinine hydrochloride; masking the odor of odorous food or drug products; and imparting acid resistance to lactic acid bacteria, enzymes, and protein based agents, although the coated foods and coated drugs according to the present invention are not limited to these uses.

In addition, aqueous shellac coating agents of the present invention are not restricted to applications for forming coatings on foodstuffs or drugs, and can also be applied to a wide variety of other applications, including electrical insulation applications (such as insulating materials for transformers, insulating varnish for use in generators or motors, insulating adhesives for use in vacuum tubes and bulbs, and for electronic processing of photoresists and the like), painting applications (such as spirit varnish for coating furniture or musical instruments, and water based paints for building materials), bonding and adhesive applications (release agents for adhesive tapes, process adhesives for gems or glass), printing applications (such as spreading agents for water based inks, and pattern paper impregnants), polishing applications (binders for felt polishing), and other applications (including cosmetic materials such as hair lacquers, moisture-proof agents for fireworks and the like, binders, and packing).

A preferred process for producing a coated food according to the present invention comprises a step in which the food is coated with a coating liquid comprising from 1 to 50% by weight of an aqueous shellac coating agent of the present invention, thereby yielding a coated food product in which the shellac solid fraction content falls within a range from 0.1 to 50% by weight of the coated food product. Similarly, a preferred process for producing a coated drug according to the present invention comprises a step in which the drug is coated with a coating liquid comprising from 1 to 50% by weight of an aqueous shellac coating agent of the present invention, thereby yielding a coated drug in which the shellac solid fraction content falls within a range from 0.1 to 50% by weight of the coated drug.

The operation of coating a coating agent of the present invention onto a foodstuff or a drug uses an aerated type pan coating apparatus or a fluidized bed coating apparatus, although the actual apparatus used is preferably selected in accordance with the formulation to be coated. In the coating operation of a coating agent of the present invention, there are no particular restrictions on the concentration of the shellac within the coating liquid, although typical values are within a range from 1 to 50% by weight, and preferably from 1 to 40% by weight, and even more preferably from 3 to 30% by weight. The shellac coating quantity can be freely altered as desired, although for foods is typically within a range from 0.1 to 50% by weight, and preferably from 0.5 to 30% by weight, and even more preferably from 1 to 15% by weight, and for drugs is preferably within a range from 0.1 to 50% by weight, and even more preferably from 0.5 to 30% by weight. For tablets, the quantity is typically within a range from 0.2 to 30% by weight, and preferably from 0.5 to 20% by weight, whereas in the case of granules, the quantity is typically within a range from 1 to 50% by weight, and preferably from 2 to 40% by weight. Furthermore, when a coating agent of the present invention is used, the target may be undercoated in advance with hydroxypropylmethylcellulose or the like, and furthermore following coating, a surface gloss agent such as wax may be overcoated on top of the coating agent of the present invention.

In another embodiment of a coated food or a coated drug according to the present invention, the food or drug is preferably covered in a multi-layered coating comprising a layer (hereafter referred to as layer A) containing an aforementioned aqueous shellac coating agent as a primary component, and a layer (hereafter referred to as layer B) containing another coating agent as a primary component. In other words, a preferred form of a coated food or a coated drug according to this embodiment is a coated food or a coated drug with a multi-layered coating comprising a layer containing an aqueous shellac coating agent as a primary component, and a layer containing another coating agent as a primary component.

In this multi-layered coating, the other coating agent that is different from the aqueous shellac coating agent is preferably formed from one or more materials selected from a group consisting of hydroxypropylmethylcellulose, methylcellulose, ethylcellulose, shellac, zein, components derived from yeast cellular walls, water soluble polysaccharides, fats, oils, waxes, and chitosan. Furthermore, in the case of drugs, the other coating agent that is different from the aqueous shellac coating agent may also contain a methacrylic acid copolymer or hydroxypropylmethylcellulose phthalate.

There are no particular restrictions on the combination of the layer A and the layer B in the multi-layered coating, and in the case of a two layer coating, the layer A can be formed as the internal layer and the layer B as the external layer, or alternatively the layer A can be formed as the external layer and the layer B as the internal layer. Furthermore, in the case of multi-layered coatings of 3 or more layers, the layer A and the layer B can be coated alternately. In such cases, the coating agent for each layer B can be either the same, or different.

Forming a B layer as an undercoat on the inside of the layer A containing aqueous shellac as the primary component performs a buffering role, and improves the stability, in those cases where, for example, the tablet components and the aqueous shellac undergo some form of interaction. By providing a coating of a B layer, formed from a coating agent that is different from the aqueous shellac coating agent, as an overcoat on the outside of the layer A containing aqueous shellac as the primary component, the B layer can be effective in masking the coloring of the tablet or the aqueous shellac, or suppressing cracking, and the A layer can be effective as a precoating for imparting water resistance and durability to the tablet.

Enteric coating agents are typically acidic materials, meaning that drugs that require enteric properties, including drugs such as benzimidazole based compounds that are easily decomposed or degenerated by the acidity, preferably do not come in direct contact with the enteric coating agent.

An aqueous shellac coating agent according to the present invention has a pH value of 6 or higher. Accordingly, even if the coating agent comes in direct contact with a drug or food that requires enteric properties, it will not cause decomposition or degeneration of the drug or food.

Accordingly, an aqueous shellac coating agent can be coated directly onto the core particles or layers of a drug or food requiring enteric properties.

Furthermore, a drug product formed from a layer of a coating liquid comprising both an aqueous shellac coating agent and a drug component represents a preferred embodiment of the present invention. Drugs requiring enteric properties can then be produced in smaller sizes with an aqueous shellac coating, and the enteric properties can also be improved.

In addition, a multi-layered coated drug comprising a layer containing an aqueous shellac coating agent and a drug component, and a layer containing a coating agent with different functions as the primary component, represents another preferred embodiment of the present invention.

Similarly, a food product comprising a layer produced from a coating liquid comprising both an aqueous shellac coating agent and a food component represents another preferred embodiment of the present invention.

Where necessary, additives such as colorants, plasticizers, masking agents, flavorings, dispersants, high viscosity polysaccharides, antioxidants, and preservatives may also be added to a coating agent of the present invention, and synthetic polymers can also be combined with the coating agent. Furthermore, in order to improve the dispersibility and prevent decomposition of these additives, a water soluble organic solvent such as ethanol, methanol, acetone, or isopropanol may also be added, although from the viewpoints of safety and environmental impact, the use of such solvents is preferably restricted to the absolute minimum.

An aqueous shellac coating agent of the present invention does not use a volatile organic solvent such as alcohol during production or during the liquid coating process, and consequently there is no danger of fire, and the safety of the working environment is excellent, and as a result the costs associated with workplace safety can be reduced. Furthermore, an aqueous shellac coating agent of the present invention does not suffer from stringiness, offers excellent coating workability, and enables a high production yield with few defects.

Furthermore, in cases where decolorized shellac is used as a raw material for coating a food or a drug with an aqueous shellac coating agent that has been produced by a production process for an aqueous shellac coating agent according to the present invention, the external appearance of the coated food or drug presents a favorable yellow or light yellow color, and the coating is also stable over time, and unlikely to degenerate.

In addition, a coating produced using such an aqueous shellac coating agent of the present invention displays excellent acid resistance, and is effective as a coating for an enteric coating, and even if the coating is immersed in an artificial gastric acid liquid (the first liquid specified in the disintegration test method of the 14th edition of the Japanese Pharmacopoeia), the swelling of the coating layer is suppressed compared with that of a shellac coating generated using sodium hydroxide, indicating an improved level of acid resistance. In other words, a coated food or a coated drug of the present invention provides excellent acid resistance together with superior intestinal juice disintegration, and is consequently effective as a food or drug requiring enteric properties.

A glazing composition for oil-based confectionary according to the present invention is in a liquid form, and comprises an aqueous shellac solution (A) containing an aqueous shellac coating agent formed from a mixture of shellac, a basic amino acid and/or a basic phosphate, a thickener (B), and/or a sugar (C). As described above, the aqueous shellac solution (A) is an aqueous solution containing an aforementioned aqueous shellac coating agent of the present invention, comprising shellac, a basic amino acid and/or a basic phosphate, and this aqueous shellac coating agent of the present invention can be either dissolved or diluted in water. This aqueous shellac solution (A) is the same as the aqueous shellac coating liquid described above.

The shellac used in the present invention can be appropriately selected from any of the conventionally available shellacs, including any of those products marketed as purified shellac, decolorized shellac or white shellac.

In the present invention, an aqueous shellac solution (A) is prepared by adding a basic amino acid and/or a basic phosphate, together with a suitable quantity of water, to the shellac. The shellac need not necessarily dissolve completely in the water, and even if residual insoluble particles of shellac remain in the solution, provided these particles are fine, they cause no significant problems during glazing of oil-based confectionary. In a preferred embodiment of the present invention, an aqueous shellac solution (A) comprises from 0.05 to 0.40 parts by weight of a basic amino acid, or from 0.04 to 0.60 parts by weight of a basic phosphate, per 1 part by weight of shellac.

The basic amino acid used is preferably one or more materials selected from a group consisting of arginine, lysine, and ornithine, although in the case of oil-based confectionary, taste acceptability means that arginine is preferably used as the sole basic amino acid. In the case in which L-arginine is used, 0.10 to 0.25 parts by weight, and preferably 0.15 to 0.18 parts by weight of L-arginine is mixed with 1 part by weight of shellac, and to 1 part by weight of this mixture is added from 0.10 to 0.95 parts by weight, and preferably from 0.23 to 0.90 parts by weight of 50 to 70°C hot water to dissolve the mixture, thereby forming an aqueous shellac solution (A). If the L-arginine content per 1 part by weight of shellac is less than 0.1 parts by weight then dissolving the shellac in the hot water becomes difficult, whereas if the content exceeds 0.25 parts by weight, although the shellac is soluble, the flavor of the L-arginine becomes overly strong, which is undesirable.

The basic phosphate can utilize those basic phosphates that are authorized for use within the production of foodstuffs or drugs, and one or more compounds selected from a group consisting of trisodium phosphate, tripotassium phosphate, disodium hydrogenphosphate, dipotassium hydrogenphosphate, tetrasodium pyrophosphate, and tetrapotassium pyrophosphate are preferred. The quantity added of the basic phosphate is typically within a range from 0.04 to 0.6 parts by weight, and preferably from 0.10 to 0.25 parts by weight, and even more preferably from 0.15 to 0.25 parts by weight, per 1 part by weight of shellac, and hot water is then preferably added to dissolve the mixture. When used for coating oil-based confectionary, taste acceptability reasons mean that from 0.15 to 0.25 parts by weight, and preferably from 0.18 to 0.22 parts by weight of disodium hydrogenphosphate is preferably added as the sole basic phosphate per 1 part by weight of shellac, and hot water is then added to dissolve the mixture and form the aqueous shellac solution (A). If the quantity of disodium hydrogenphosphate is less than 0.15 parts by weight, then dissolving the shellac becomes somewhat difficult, whereas if the quantity exceeds 0.25 parts by weight, the alkali taste becomes overly strong, which is undesirable from an acceptability viewpoint.

The quantity of water (preferably hot water) added to an aqueous shellac coating agent containing a mixture of shellac and a basic amino acid, and/or a basic phosphate is preferably sufficient to produce a concentration of the aqueous shellac coating agent within the resulting aqueous shellac solution (A) of 1 to 40% by weight, and even more preferably from 10 to 30% by weight. If the aqueous shellac coating agent accounts for less than 1% by weight, then the quantity of shellac in a glazing composition of the present invention, produced by combining this aqueous shellac solution (A) with a thickener (B) and/or a sugar (C), is insufficient, resulting in an unfavorable reduction in the holding power of the glaze to the surface of oil-based confectionary. In contrast, if the quantity of the aqueous shellac coating agent exceeds 40% by weight, then the viscosity of a glazing composition of the present invention produced by combining the aqueous shellac solution (A) with a thickener (B) and/or a sugar (C) becomes overly high, and applying the glazing composition to the surface of oil-based confectionary becomes overly difficult. Furthermore, the temperature of the hot water used for dissolving the mixture of shellac, the basic amino acid and/or the basic phosphate is preferably set within a range from 50 to 70°C. If the temperature of the hot water is less than 50°C, then the mixture cannot be readily dissolved, which is undesirable. In contrast, if the temperature of the hot water exceeds 70°C, the shellac can degenerate, leading to an undesirable deterioration in the film forming capabilities of the coating agent.

In those cases where an aqueous shellac solution (A) described above is applied directly to the surface of oil-based confectionary, the aqueous shellac solution (A) penetrates into the oil-based confectionary, and is unable to form a glaze-like coating on the surface of the confectionary, meaning an attractive glaze with a good level of gloss cannot be obtained.

As a result of intensive investigations, the inventors of the present invention discovered that by adding a thickener (B) and/or a sugar (C) to the aqueous shellac solution (A), an effective glaze could be imparted to the surface of oil-based confectionary.

Examples of suitable thickeners (B) that can be added to an aqueous shellac solution (A) include either one, or a mixture of two or more materials selected from a group consisting of pullulan, xanthan gum, guar gum, locust bean gum, tamarind gum, pectin, carrageenan, tragacanth gum, gum arabic, gelatin, and collagen. Of these, pullulan, xanthan gum and guar gum are preferred. These thickeners (B) may be either dissolved in the aqueous shellac solution (A), or an aqueous solution containing either the thickener (B) or a mixture of the thickener (B) and a sugar (C) dissolved in hot water may be mixed with the aqueous shellac solution (A). The quantity added of this type of thickener (B) is preferably equivalent to 1 to 10% by weight, and even more preferably 4 to 9% by weight, of the glazing composition of the present invention. If the quantity of the thickener (B) is less than the above range, then producing an attractive glaze with a good level of gloss becomes difficult. In contrast, if the quantity of the thickener (B) exceeds the above range, the viscosity of the glazing composition becomes overly high, making the glazing operation more difficult.

Examples of the sugar (C) added to the aqueous shellac solution (A) include either one, or a mixture of two or more materials selected from a group consisting of monosaccharides, disaccharides, oligosaccharides, acid-saccharified starch syrup, enzyme-saccharified starch syrup, and starch decomposition products. Of these, sucrose, liquid sugar of fructose-glucose mixtures, starch decomposition products of no more than 45 dextrin equivalents, acid-saccharified starch syrup, and enzyme-saccharified starch syrup are preferred. In those cases when a sugar (C) is added, the sugar concentration within the glazing composition is preferably set within a range from 8 to 80% by weight. At sugar concentrations of less than 8% by weight, the durability of the glaze produced by applying the glazing composition to the surface of oil-based confectionary is undesirably poor, although the technical reasons for this phenomenon remain unclear. In contrast, if the sugar concentration exceeds 80% by weight, the viscosity of the glazing composition becomes overly high, making application of the composition to the surface of oil-based confectionary difficult, and effectively preventing the formation of an attractive glaze.

A sugar alcohol may also be added to the aqueous shellac solution (A) instead of the sugar (C). Suitable examples of the sugar alcohol for addition to the aqueous shellac solution (A) include one or more compounds selected from a group consisting of reduced starch syrup, sorbitol, maltitol, and xylitol. In those cases when a sugar alcohol is added, the sugar alcohol concentration within the glazing composition is preferably set within a range from 8 to 80% by weight. At sugar alcohol concentrations of less than 8% by weight, the durability of the glaze produced by applying the glazing composition to the surface of oil-based confectionary is unsatisfactory and undesirable. In contrast, if the sugar alcohol concentration exceeds 80% by weight, the viscosity of the glazing composition becomes overly high, making application of the composition to the surface of oil-based confectionary difficult, and effectively preventing the formation of an attractive glaze.

The sugar alcohol exhibits the same functions as the sugar (C) within the glazing composition, and if required a combination of a sugar (C) and a sugar alcohol can also be used. In such a case, the combined weight of the sugar (C) and the sugar alcohol preferably falls within the concentration range described above.

A glazing composition of the present invention comprises an aqueous shellac solution (A), a thickener (B) and/or a sugar (C), and the quantities of the aqueous shellac solution (A), the thickener (B), the sugar (C), and any added water are adjusted to ensure that the composition is in liquid form. The quantity of the aqueous shellac solution (A) should be adjusted so as to produce a concentration of the aqueous shellac coating agent within the product glazing composition of 0.1 to 30% by weight, and preferably from 1 to 25% by weight, and even more preferably 3 to 20% by weight, and most preferably from 5 to 15% by weight. If the concentration of the aqueous shellac coating agent within the glazing composition is less than the lower limit of the above range, then the glazing effect on the surface of the oil-based confectionary is unsatisfactory. In contrast, if the concentration of the aqueous shellac coating agent exceeds the upper limit of above range, the viscosity of the glazing composition becomes overly high, making application of the composition to the surface of oil-based confectionary difficult, and effectively preventing the formation of an attractive glaze.

A preferred embodiment of a glazing composition of the present invention comprises an aqueous shellac solution (A), a thickener (B), and/or a sugar (C) as described above, is in a liquid form, and contains essentially no organic solvents. Because a glazing composition of the present invention enables an attractive glaze to be imparted to the surface of oil-based confectionary without requiring the use of organic solvents, safety during production can be improved, and any deleterious impact on the environment can be prevented.

As follows is a description of a process for glazing oil-based confectionary according to the present invention.

In a glazing process according to the present invention, a glazing composition which is in a liquid form and comprises an aforementioned aqueous shellac solution (A), a thickener (B), and/or a sugar (C) is applied to oil-based confectionary to be glazed, and is then dried while being polished if required.

In a glazing process of the present invention, suitable examples of the oil-based confectionary to be glazed include granular oil-based confectionary (also known as dragee) comprising one or more types of confectionary selected from a group consisting of chocolate, white chocolate and nut cream. Specific examples of these types of granular oil-based confectionary include granular confectionary produced by coating edible granules such as chocolate, oil-based cream, nuts (such as almonds, macadamia nuts, peanuts, hazel nuts, cashew nuts and walnuts) or candy with a material such as oil-based cream, chocolate or white chocolate, and performing subsequent molding.

The quantity of the glazing composition relative to that of the oil-based confectionary being coated is preferably within a range from 0.05 to 5 parts by weight, and even more preferably from 0.2 to 1 part by weight per 100 parts by weight of the oil-based confectionary. If the quantity of the glazing composition is less than the above range, then the glazing on the oil-based confectionary is inadequate. In contrast, if the quantity of the glazing composition exceeds the above range, the time required for the glazing treatment, and particularly the drying time, become overly long, causing an undesirable worsening of the productivity.

A glazing process of the present invention can be realized simply and quickly by adding and applying a glazing composition while the oil-based confectionary is rolled within a rotary pan, and subsequently subjecting the glazed confectionary to forced-air drying. The rotary pan used can utilize a conventional rotary pan or an aerated drum type rotary pan such as those typically used in the fields of food production (and particularly the production of granular confectionary) or drug production (and particularly the production of pills and sugar coated tablets), and the glazing composition is preferably either added dropwise or sprayed into the rotary pan.

By adding the glazing composition while the oil-based confectionary is rolled inside the rotary pan, the glazing composition bonds in a thin, uniform layer to the surface of the oil-based confectionary, forming a thin coating containing shellac on the surface of the confectionary. By applying the glazing composition and then rolling the oil-based confectionary inside the rotary pan, the surfaces of the oil-based confectionary granules rub against each other in a polishing action, meaning an attractive glaze with a good level of gloss can be achieved without the need for a separate polishing treatment.

The oil-based confectionary within the rotary pan is subjected to forced-air drying, either during the addition of the glazing composition, or following completion of the addition and after conducting rolling of the confectionary for a specified time. The forced-air drying is conducted under conditions that enable satisfactory drying of the glazing composition while ensuring that the oil-based confectionary does not melt. For example, the forced-air drying can be conducted by blowing dried air at 10 to 20°C and with a relative humidity of 25 to 65% into the rotary pan until moist air generated from the applied glazing composition ceases to be produced. Following drying, the glazed oil-based confectionary is transported to a filling and packaging process, and is packaged within a suitable container to complete the production of the product.

As described above, a glazing process of the present invention uses a glazing composition that contains essentially no organic solvents, and similarly, it is preferred that essentially no organic solvents be used within the glazing treatment. Using a glazing process of the present invention, an attractive glaze can be formed on the surface of oil-based confectionary without using organic solvents, and consequently safety during production can be improved, and any deleterious impact on the environment can be prevented.

Glazed oil-based confectionary of the present invention has undergone a glazing treatment on the surface of the confectionary using the glazing process described above, and consequently an attractive glaze can be provided on the surface of the oil-based confectionary without using organic solvents. The present invention also provides glazed oil-based confectionary obtainable using the process for glazing oil-based confectionary described above.

### EXAMPLES

As follows is a more detailed description of the present invention based on a series of examples, although the present invention is in no way restricted to these examples.

### [Example 1]

### - Preparation of a Coating Liquid

10 parts by weight of decolorized shellac was dispersed in 88.35 parts by weight of distilled water at 55°C, and with the mixture undergoing constant stirring with a stirrer, 1.65 parts by weight of L-arginine was added, the resulting mixture was stirred thoroughly until no large particles remained within the liquid, and nitrogen gas was then bubbled through the liquid until the residual dissolved oxygen concentration within the liquid was no more than 2 mg/L, thereby completing the preparation of a coating liquid (containing 10% by weight of shellac) for a coating agent of the present invention.

### - Preparation of Coated Tablets

350 g of white triangular tablets with a weight of 220 mg per tablet were set in a coating apparatus (brand name "Hicoater lab", manufactured by Freund Industrial Co., Ltd.), and using operating conditions including an air supply temperature of 52°C, an air supply rate of 0.5 m³/minute, a spray rate of 2 g/minute, a spray pressure of 0.1 MPa, and a pan rotational speed of 20 rpm, the triangular tablets were sprayed with the coating liquid described above until the shellac solid fraction reached a value of 12% by weight of the total tablet weight, thereby yielding coated tablets.

### [Example 2]

With the exceptions of altering the quantity of distilled water to 88.4 parts by weight, and using 1.6 parts by weight of tetrasodium pyrophosphate instead of the L-arginine, a coating liquid of the present invention was prepared in the same manner as the example 1. The same coating operation as the example 1 was then conducted, yielding coated tablets in which the shellac solid fraction was 12% by weight of the total tablet weight.

### [Example 3: Preparation of Taste Masking Granules]

500 g of granules containing 5.5% by weight of a bitter tasting thiamine hydrochloride (granule diameter 12 to 32 mesh) were set in a fluidized bed granule coating apparatus (brand name "Flow Coater lab", manufactured by Freund Industrial Co., Ltd.), and using the same coating liquid as the example 1, and under conditions including an air supply temperature of 70°C, an air supply rate of 0.5 m³/minute, a spray rate of 3 g/minute, and a spray pressure of 0.15 MPa, coated granules were obtained in which the shellac solid fraction was 7% by weight of the total granule weight.

### [Example 4: Moisture Permeability Test]

The coating liquid prepared in the example 1 was dried on top of a flat Schale formed from a resin (at a temperature of 50°C), yielding a casting film of thickness 90 µm. The moisture permeability of this film obtained from the coating liquid of the example 1 was then measured in accordance with the test method specified in the Japan Industrial Standards (JIS Z0208).

### [Comparative Example 1]

With the exceptions of altering the quantity of distilled water to 89.4 parts by weight, and using 0.6 parts by weight of sodium hydroxide instead of the L-arginine, a coating liquid was prepared in the same manner as the example 1. The same coating operation as the example 1 was then conducted, yielding coated tablets in which the shellac solid fraction was 12% by weight of the total tablet weight.

### [Comparative Example 2]

10 parts by weight of decolorized shellac, 2.5 parts by weight of vegetable oil (hardened palm oil), and 2.3 parts by weight of the monoglycerol ester of oleic acid were added to 85.2 parts by weight of ethanol, and the resulting mixture was stirred until a transparent solution was obtained, thereby completing the preparation of a coating solution. Using the same apparatus as the example 1, coating was conducted under operating conditions including an air supply temperature of 38°C, an air supply rate of 0.5 m³/minute, a spray rate of 2 g/minute, a spray pressure of 0.1 MPa, and a pan rotational speed of 20 rpm, yielding coated tablets in which the shellac solid fraction was 12% by weight of the total tablet weight.

### [Comparative Example 3]

With the exception of altering the shellac solid fraction coated onto the tablets to a value of 6% by weight relative to the tablet weight, coated tablets were prepared using the same operation as the comparative example 2.

### [Comparative Example 4]

With the exception of using an 8% by weight aqueous solution of hydroxypropylmethylcellulose as the coating liquid, a casting film of thickness 90 µm was prepared, and the moisture permeability was measured, in the same manner as the example 4.

### [Comparison of Coating Characteristics]

For the above examples 1 and 2, and the comparative examples 1 to 3, the methods described below were used to evaluate the coating workability, and the coloring, the acid resistance, the disintegration in intestinal juices, and the stability of the coating on the coated tablets. The results are shown in Table 1.

### <Coating Workability>

In each coating operation, the tablets were inspected for the presence of adhesion of the tablets to the coating pan due to stringiness of the coating liquid, and peeling of the coating at the coated surface, and were then evaluated using the following criteria.
○ No coating faults. Coated tablets with a uniform coating were obtained.
Δ Some coating faults. The surface coating had peeled away in some tablets.
× Coating faults: Tablets adhered to the coating pan, and the surface coating had peeled away in most tablets.

### <Coloring of the Coating>

The external coloring of each of the coated tablets was inspected visually. The color of the surface coating on the tablets was recorded.

### <Disintegration Tests: Determination of Gastric Juice Resistance and Intestinal Juice Disintegration>

Each of the coated tablets was evaluated in accordance with the test method for enteric formulations, one of the disintegration test methods (B-619) detailed in the 14th edition of the Japanese Pharmacopoeia. The first liquid used as a test liquid corresponds with artificial gastric juice, and was used to evaluate the acid resistance of the coating, whereas the second liquid corresponds with artificial intestinal juice, and was used to evaluate the disintegration of the coating within the intestine.

In the tests using the first liquid, the dissolution or disintegration of the coating was determined and the permeation of the first liquid into the coated tablets was viewed, and was evaluated using the following criteria.
○ Two hours after commencing the disintegration test, there were no marked changes in the coated tablets.
× Two hours after commencing the disintegration test, swelling and/or disintegration of the coated tablets resulting from permeation of the first liquid was marked.

Furthermore, in the tests using the second liquid, the time required to reach the standard for intestinal disintegration was measured.

### <Stability Test>

Each of the coated tablets was packaged in PTP and stored for 3 months in an atmosphere at 40°C, and the above disintegration tests were then conducted to evaluate the stability of the tablets. The evaluation method used was the same as that described above for the disintegration tests.

**Table 1**

| | | Example 1 | Example 2 | Comparative Example 1 | Comparative Example 2 | Comparative Example 3 |
|---|---|---|---|---|---|---|
| Coating workability | | ○ | ○ | × adhesion to pan, peeling of coating | Δ adhesion to pan, peeling of coating | Δ adhesion to pan, peeling of coating |
| Coating coloring | | cream | light cream | light brown | cream | light cream |
| Disintegration tests | First liquid (gastric acid resistance) | ○ | ○ | × marked swelling or disintegration | ○ | × marked swelling or disintegration |
| | Second liquid (intestinal juice disintegration) | within 10 minutes | within 10 minutes | within 10 minutes | did not disintegrate | 50 minutes |
| Stability Test | First liquid (gastric acid resistance) | ○ | ○ | × marked swelling or disintegration | ○ | × marked swelling or disintegration |
| | Second liquid (intestinal juice disintegration) | within 10 minutes | within 10 minutes | within 10 minutes | did not disintegrate | did not disintegrate |

From the results shown in Table 1, it is clear that the examples 1 and 2, which utilize aqueous shellac coating agents of the present invention, display excellent coating workability with no stringiness, and provide a high product yield with few defects, when compared with both the comparative example 1, which represents a conventional aqueous coating agent prepared using sodium hydroxide, and the comparative examples 2 and 3, which utilize coating agents in which the shellac is dissolved in an organic solvent (ethanol).

Furthermore, the colorings of the coatings from the examples 1 and 2 are lighter than that of the comparative example 1, and provide a favorable external appearance.

In addition, the coatings of the examples 1 and 2 display sufficiently favorable levels of gastric acid resistance and enteric disintegration to enable their practical application within enteric coatings.

### [Comparison of Masking Performance of Coatings]

The coated granules produced in the example 3, and uncoated granules were evaluated for taste masking effect using the method described below. The results are shown in Table 2.

### <Evaluation of Taste Masking Effect>

Using the coated granules produced in the example 3 and uncoated granules, taste masking was evaluated using a sensory test. 0.2 g of granules were placed on the tongue, and the time taken to notice a bitter taste was measured for five panelists. The average of the five times was then calculated and recorded.

**Table 2**

| | Example 3 | Uncoated granules |
|---|---|---|
| Time taken to notice bitter taste | 47.5 seconds | 2.5 seconds |

From the results shown in Table 2, it is clear that the coated granules of the example 3 of the present invention display a much longer time for the bitter taste to be noticed than the uncoated granules, indicating that the coating agent of the present invention has a satisfactory taste masking effect.

### [Comparison of Moisture Resistance of Coatings]

Using the casting films prepared in the example 4 and the comparative example 4, the moisture permeability was measured in accordance with the moisture permeability test of JIS Z0280. The test conditions used were (1) 25°C, relative humidity 92%, and (2) 40°C, relative humidity 89%, enabling the moisture permeability (units: g/m²·24hr) of each film to be evaluated. The results are shown in Table 3.

**Table 3**

| | Example 4 | Comparative Example 4 |
|---|---|---|
| 25°C, relative humidity 92% | 155 | 818 |
| 40°C, relative humidity 89% | 436 | 1361 |
| units: g/m²·24hr | | |

From the results shown in Table 3, it is clear that the coating of the example 4 according to the present invention displays a lower level of moisture permeability and a superior level of moisture resistance to the coating of the comparative example 4 formed from hydroxypropylmethylcellulose.

### [Investigation of the Required Quantity of Basic Amino Acid and/or Basic Phosphate]

Using arginine as the basic amino acid and tetrasodium pyrophosphate as the basic phosphate, the quantity of each of these materials required to form an aqueous coating agent with each of the various types of shellac was investigated.

Using decolorized shellac (acid value 73.4) and white shellac (acid value 84.0) as the shellac samples, the quantity of base required to form an aqueous solution of 1 part by weight of the shellac was determined.

In the case of arginine, 0.15 to 0.17 parts by weight were required to generate an aqueous solution with 1 part by weight of decolorized shellac, whereas with white shellac, this quantity increased to 0.21 to 0.25 parts by weight.

Furthermore in the case of tetrasodium pyrophosphate, 0.14 to 0.18 parts by weight were required to generate an aqueous solution with 1 part by weight of decolorized shellac, whereas with white shellac, this quantity increased to 0.20 to 0.26 parts by weight.

As shown above, the quantity of base required to form an aqueous solution of the shellac was different for the decolorized shellac and the white shellac. This difference is caused by the different shellac production processes, and is due mainly to the different acid values generated as a result of the production process. Because shellac is a natural product, the standards relating to acid value recorded in Japan's Specifications and Standards for Food Additives and the Japanese Pharmacopoeia are comparatively broad. The reason for this broadness is to allow for variations in quality of the raw material, and consequently there is a possibility that the predetermined quantities of base determined in the above tests will be either excessive or insufficient (particularly, insufficient). Accordingly, the above required quantity ranges for the basic amino acid (arginine) and the basic phosphate (tetrasodium pyrophosphate) were corrected to ensure that the standard ranges for the shellac acid value could be covered.

According to these corrected ranges, in the case of arginine, 0.12 to 0.19 parts by weight are required to generate an aqueous solution with 1 part by weight of decolorized shellac, whereas with white shellac the range is from 0.16 to 0.29 parts by weight. In the case of tetrasodium pyrophosphate, 0.12 to 0.22 parts by weight are required to generate an aqueous solution with 1 part by weight of decolorized shellac, whereas with white shellac the range is from 0.18 to 0.28 parts by weight.

These addition quantities of basic amino acid and basic phosphate refer to the addition quantities for arginine and tetrasodium pyrophosphate relative to refined decolorized shellac or white shellac, and if a basic amino acid other than arginine, or a basic phosphate other than tetrasodium pyrophosphate is used, then the ideal addition quantity will vary. Furthermore, aqueous shellac coating agents of the present invention include not only solutions in which the shellac is completely dissolved, but also shellac dispersions in which a portion of the shellac is dissolved and the remainder is dispersed in the form of undissolved fine particles. When this type of dispersion coating liquid is prepared, the quantity added of the basic amino acid and/or the basic phosphate may be lower than the lower limit of the above quantity ranges. Taking these cases into consideration, the quantity of the basic amino acid added can be within a range from 0.05 to 0.40 parts by weight per 1 part by weight of the shellac, and the quantity of the basic phosphate added can be within a range from 0.04 to 0.60 parts by weight per 1 part by weight of the shellac.

### [Example 5]

With the exception of altering the quantity of distilled water to 85.75 parts by weight, a coating liquid was prepared in the same manner as the example 1. 0.6 parts by weight of glycerol and 2 parts by weight of a sucrose fatty acid ester (HLB 6) were then added to the liquid, and the resulting mixture was stirred thoroughly until no large particles remained, thereby yielding a coating liquid for a coating agent of the present invention. The same coating operation as the example 1 was then conducted, yielding coated tablets in which the shellac solid fraction was 8% by weight of the total tablet weight.

### [Example 6]

Using the same coating operation as the example 1, an 8% by weight aqueous solution of hydroxypropylmethylcellulose was sprayed onto the coated tablets produced in the example 5 until the hydroxypropylmethylcellulose solid fraction reached a value of 3% by weight of the total tablet weight, thereby yielding multi-layered coated tablets comprising a coating of hydroxypropylmethylcellulose as an overcoat on the outside of the aqueous shellac coating layer.

### [Cracking Resistance of Coatings]

Each of the coated tablets from the examples 5 and 6 and the comparative example 1 was placed in a glass bottle together with a desiccant (silica gel), and the bottle was then sealed and stored, and the tablets were inspected for evidence of cracking. The test conditions included a temperature of 25°C for a period of 10 days. The results are shown in the table below.

**Table 4**

| | Example 5 | Example 6 | Comparative example 1 |
|---|---|---|---|
| Surface condition of tablets | No cracking | No cracking | Cracking |

From the results shown in Table 4, it is clear that the coatings of the examples 5 and 6 according to the present invention display superior cracking resistance (cracking suppression) even under dry conditions

### [Example 7]

500 g of spherical granules formed from sucrose and corn starch (brand name: Nonpareil 101, manufactured by Freund Industrial Co., Ltd.), with a granule diameter of 22 to 30 mesh, were set in a fluidized bed granule coating apparatus (brand name "Flow Coater lab", manufactured by Freund Industrial Co., Ltd.), and under conditions including an air supply temperature of 65°C, an air supply rate of 0.5 m³/minute, a spray rate of 3 g/minute, and a spray pressure of 0.15 MPa, a mixed liquid containing an aqueous shellac coating agent and the digestive enzyme agent pancreatin in the relative proportions shown in Table 5 was sprayed onto the granules, yielding coated granules in which the shellac solid fraction represented 25% by weight, and the pancreatin represented 10% by weight, of the total granule weight.

**Table 5**

| | |
|---|---|
| Aqueous shellac coating liquid prepared in the example 1 | 100 parts by weight |
| Pancreatin | 4 parts by weight |

### [Evaluation of Enteric Properties of Coated Granules]

The enteric properties of the coated granules prepared in the example 7 were evaluated. The evaluation method used was the same as that described above in the subsection entitled <Disintegration Tests: Determination of Gastric Juice Resistance and Intestinal Juice Disintegration> within the section comparing the coating characteristics of the examples 1 and 2 and the comparative examples 1 to 3. The results are shown in Table 6.

**Table 6**

| | Example 7 |
|---|---|
| First liquid (gastric acid resistance) | ○ |
| Second liquid (intestinal juice disintegration) | within 15 minutes |

From the results shown in Table 6, it is clear that the coated granules of the example 7, containing pancreatin within the coating, display sufficiently favorable levels of gastric juice resistance and intestinal juice disintegration to enable their practical application within enteric coatings.

### [Example 8]

16.5 parts by weight of L-arginine ("L-arginine RS", manufactured by Kyowa Hakko Kogyo Co., Ltd.) was mixed with 100 parts by weight of purified shellac powder (purified shellac, manufactured by Gifu Shellac Manufacturing Co., Ltd.), and to 30 parts by weight of this mixture was added 70 parts by weight of hot water at 70°C, thereby dissolving the mixture and yielding 100 parts by weight of an aqueous shellac solution (A).

To 20 parts by weight of this aqueous shellac solution (A) were added 20 parts by weight of acid-saccharified starch syrup (38 Starch syrup, manufactured by Sanmatsu Kogyo Co., Ltd.) and 30 parts by weight of sucrose as the sugar (C), and dissolution of the sugar (C) yielded a liquid glazing composition with a sugar concentration of 64.4% by weight and a shellac concentration of 7.4% by weight.

1500 g of almond chocolate balls with a uniform coating of chocolate provided on the surface of each almond granule and with a weight of 4 g /granule were placed in a rotary pan (FM-2, manufactured by Freund Industrial Co., Ltd., a fully automatic film coating apparatus with a stainless steel barrel of which diameter is 300mm), and with the rotary pan undergoing rotation at 35 rpm, 3 g of the glazing composition of the present invention prepared in the manner described above was applied to the surface of the almond chocolate balls.

Subsequently, air at a temperature of 20°C and a relative humidity of 50% was blown onto the surface of the almond chocolate balls, which were still being rolled around inside the pan, thereby removing the moisture and drying the glazing composition.

The above operation was repeated 3 times, yielding almond chocolate balls with an attractive glaze on the surface.

Following storage for 3 days at 23°C and 60% humidity, the glazed almond chocolate balls were subjected to a durability test by placing the chocolate balls in a thermostatic chamber at 25°C and 70% humidity for 24 hours. Inspection of the glaze after the 24 hour period revealed that in comparison with the comparative example 5 described below, which was treated with a composition containing no shellac, the glaze of this example displayed good durability, and suffered no loss of glaze, nor stickiness.

Furthermore, in this example 8 of the present invention, a durable glaze can be applied to oil-based confectionary without requiring the use of organic solvents such as ethanol or isopropanol, meaning concerns of atmospheric pollution by volatile organic matter do not arise.

### [Comparative Example 5]

With the exception of replacing the 20 parts by weight of the aqueous shellac solution (A) used in the example 8 with 20 parts by weight of a 30% by weight aqueous solution of sucrose, treatment was conducted in the same manner as the example 8, yielding glazed almond chocolate balls. The sugar concentration of the glazing composition of the comparative example 5, which contained no shellac, was 70.4%.

The almond chocolate balls from the comparative example 5 were stored and then subjected to a durability test under the same conditions as the example 8, although in comparison with the product from the example 8, the chocolate balls showed a loss of glaze as well as stickiness, and were unattractive products likely to result in a loss of commercial value.

The products obtained in the example 8 and the comparative example 5 were placed in a Schale, and then allowed to stand in a thermostatic chamber at 25°C and 70% humidity. The results of inspecting the state of each product at 1 hourly intervals are summarized in Table 7.

**Table 7**

| | Example 8 | | Comparative Example 5 | |
|---|---|---|---|---|
| Time elapsed | Glaze evaluation | Stickiness evaluation | Glaze evaluation | Stickiness evaluation |
| Start | Θ | Θ | Θ | ○ |
| 5 hours elapsed | Θ | Θ | Θ | Δ |
| 10 hours elapsed | Θ | Θ | ○ | × |
| 15 hours elapsed | Θ | Θ | Δ | × |
| 20 hours elapsed | Θ | ○ | × | × |
| 24 hours elapsed | Θ | Δ | × | × |

In FIG. 7, the grades recorded for "glaze evaluation" were determined by inspecting the almond chocolate product for the presence of glaze, and for discoloration of that glaze, and then assigning a grade based on the following criteria.
- Θ: No change from the original state.
- ○: Some glaze lost, although retains commercial value.
- Δ: A little glaze left, but significant reduction in commercial value.
- ×: No glaze, and no commercial value.

Furthermore, the grades recorded for "stickiness evaluation" were determined by inspecting the almond chocolate product for surface stickiness, and then assigning a grade based on the following criteria. The evaluation grades were determined on the basis of finger contact with the product surface.
- Θ: Almost no stickiness, essentially unchanged from the original state.
- ○: Some stickiness, although retains commercial value.
- Δ: Sticky, with significant reduction in commercial value.
- ×: Very sticky, with no commercial value.

From the results shown in Table 7, it is evident that the product of the example 8, which has been glazed with a glazing composition according to the present invention, enables good retention of the glaze over extended periods, and suffers only minor stickiness.

### [Example 9]

40 parts by weight of the aqueous shellac solution (A) prepared in the example 8, 10 parts by weight of Sandek (Sandek #30, manufactured by Sanwa Cornstarch Co., Ltd.), 20 parts by weight of sucrose, and 30 parts by weight of hot water at 60°C were mixed together, and on dissolution yielded a glazing composition with a sugar concentration of 29% by weight, and a shellac concentration of 10.3% by weight.

Using this glazing composition, an almond chocolate product was glazed in the same manner as described in the example 8, and the glazed product was then subjected to the same durability test as the example 8. The results revealed that for the product of this example 9, the surface glaze displayed good durability, and there was no loss of glaze, nor stickiness.

### [Example 10]

50 parts by weight of the aqueous shellac solution (A) prepared in the example 8, 16 parts by weight of pullulan (Pullulan PF20, manufactured by Hayashibara Group), and 34 parts by weight of hot water at 60°C were mixed together, and on dissolution yielded a glazing composition with a sugar concentration of 8% by weight, and a shellac concentration of 12.9% by weight.

Using this glazing composition, an almond chocolate product was glazed in the same manner as described in the example 8, and the glazed product was then subjected to the same durability test as the example 8. The results revealed that for the product of this example 10, the surface glaze displayed good durability, and there was no loss of glaze, nor stickiness.

### [Example 11]

16.5 parts by weight of L-arginine ("L-arginine RS", manufactured by Kyowa Hakko Kogyo Co., Ltd.) was mixed with 100 parts by weight of purified shellac powder (purified shellac, manufactured by Gifu Shellac Manufacturing Co., Ltd.), and to 10 parts by weight of this mixture was added 90 parts by weight of hot water at 70°C, thereby dissolving the mixture and yielding 100 parts by weight of an aqueous shellac solution (A).

50 parts by weight of this aqueous shellac solution (A), 13 parts by weight of the starch decomposition product "Pineflow" (manufactured by Matsutani Chemical Industry Co., Ltd.), and 37 parts by weight of hot water at 60°C were mixed together, and on dissolution yielded an aqueous shellac glazing composition, with a sugar concentration of 13% by weight, and a shellac concentration of 4.3% by weight.

Using this glazing composition, an almond chocolate product was glazed in the same manner as described in the example 8, and the glazed product was then subjected to the same durability test as the example 8. The results revealed that for the product of this example 11, the surface glaze displayed good durability, and there was no loss of glaze, nor stickiness.

## Claims

1. An aqueous shellac coating agent comprising shellac, and a basic amino acid and/or a basic phosphate.

2. An aqueous shellac coating agent according to claim 1, wherein said basic amino acid is one or more materials selected from a group consisting of arginine, lysine, and ornithine.

3. An aqueous shellac coating agent according to claim 1, wherein said basic phosphate is one or more materials selected from a group consisting of trisodium phosphate, tripotassium phosphate, disodium hydrogenphosphate, dipotassium hydrogenphosphate, tetrasodium pyrophosphate, and tetrapotassium pyrophosphate.

4. An aqueous shellac coating agent according to claim 1, wherein a quantity of said basic amino acid is within a range from 0.05 to 0.40 parts by weight per 1 part by weight of said shellac.

5. An aqueous shellac coating agent according to claim 2, wherein a quantity of said basic amino acid is within a range from 0.05 to 0.40 parts by weight per 1 part by weight of said shellac.

6. An aqueous shellac coating agent according to claim 1, wherein a quantity of said basic phosphate is within a range from 0.04 to 0.60 parts by weight per 1 part by weight of said shellac.

7. An aqueous shellac coating agent according to claim 3, wherein a quantity of said basic phosphate is within a range from 0.04 to 0.60 parts by weight per 1 part by weight of said shellac.

8. An aqueous shellac coating agent according to claim 1, further comprising one or more materials selected from a group consisting of aliphatic polyols, fatty acid esters, water soluble sugars, triethyl citrate, polyethylene glycol, and sodium lactate.

9. An aqueous shellac coating agent according to claim 8, wherein said aliphatic polyol is one or more compounds selected from a group consisting of glycerol, propylene glycol, and sugar alcohols.

10. An aqueous shellac coating agent according to claim 9, wherein said sugar alcohol is one or more compounds selected from a group consisting of sorbitol, maltitol, erythritol, xylitol, mannitol, palatinit, and lactitol.

11. An aqueous shellac coating agent according to claim 8, wherein said fatty acid ester is one or more compounds selected from a group consisting of sucrose fatty acid esters, mono-, di-, tri- and polyglycerol fatty acid esters, organic acid monoglycerides, propylene glycol fatty acid esters, sorbitan fatty acid esters, and polysorbates.

12. An aqueous shellac coating agent according to claim 8, wherein said water soluble sugar is one or more compounds selected from a group consisting of trehalose, oligosaccharides, maltose, galactose, lactose, sucrose, glucose, and fructose.

13. A process for producing an aqueous shellac coating agent, comprising the steps of mixing shellac with a basic amino acid solution, a basic phosphate solution, or a mixed solution of a basic amino acid and a basic phosphate, preparing an aqueous shellac coating liquid with said shellac stably dissolved or dispersed therein, and then either concentrating or drying said liquid, or subjecting said liquid to neither concentration nor drying.

14. A process for producing an aqueous shellac coating agent, comprising the steps of dispersing shellac in a solution of an acidic material, subsequently adding a basic alkali metal salt to said solution, preparing an aqueous shellac coating liquid with said shellac stably dissolved or dispersed therein, and then either concentrating or drying said liquid, or subjecting said liquid to neither concentration nor drying.

15. A process for producing an aqueous shellac coating agent according to claim 14, wherein said basic alkali metal salt is one or more compounds selected from a group consisting of alkali metal hydroxides, carbonates, and bicarbonates.

16. A process for producing an aqueous shellac coating agent according to claim 14, wherein said acidic material is one or more compounds selected from a group consisting of phosphoric acid and polyphosphoric acid.

17. A process for producing an aqueous shellac coating agent according to any one of claim 13 through claim 16, comprising an inert gas treatment step for passing an inert gas through said aqueous shellac coating liquid and replacing any gas within said liquid.

18. A process for producing an aqueous shellac coating agent according to claim 17, wherein said inert gas is one or more gases selected from a group consisting of nitrogen, argon, and helium.

19. A coated food formed by coating a food with an aqueous shellac coating agent according to any one of claim 1 through claim 12.

20. A coated food with a multi-layered coating comprising a layer containing an aqueous shellac coating agent according to any one of claim 1 through claim 12 as a primary component, and a layer containing another coating agent as a primary component.

21. A coated food according to claim 20, wherein said other coating agent is formed from one or more materials selected from a group consisting of hydroxypropylmethylcellulose, methylcellulose, ethylcellulose, shellac, zein, components derived from yeast cellular walls, water soluble polysaccharides, fats, oils, waxes, and chitosan.

22. A process for producing a coated food, comprising a step for coating a food with a coating liquid containing from 1 to 50% by weight of an aqueous shellac coating agent according to any one of claim 1 through claim 12, wherein a shellac solid fraction content within a produced coated food is within a range from 0.1 to 50% by weight.

23. A coated drug formed by coating a drug with an aqueous shellac coating agent according to any one of claim 1 through claim 12.

24. A coated drug formed by coating a drug with a coating agent containing an aqueous shellac coating agent according to any one of claim 1 through claim 12 and a drug component.

25. A coated drug with a multi-layered coating comprising a layer containing an aqueous shellac coating agent according to any one of claim 1 through claim 12 as a primary component, and a layer containing another coating agent as a primary component.

26. A coated drug with a multi-layered coating comprising a layer containing an aqueous shellac coating agent according to any one of claim 1 through claim 12 and a drug component, and a layer containing another coating agent as a primary component.

27. A coated drug according to claim 25, wherein said other coating agent is formed from one or more materials selected from a group consisting of methacrylic acid copolymers, hydroxypropylmethylcellulose, hydroxypropylmethylcellulose phthalate, methylcellulose, ethylcellulose, shellac, zein, components derived from yeast cellular walls, water soluble polysaccharides, fats, oils, waxes, and chitosan.

28. A coated drug according to claim 26, wherein said other coating agent is formed from one or more materials selected from a group consisting of methacrylic acid copolymers, hydroxypropylmethylcellulose, hydroxypropylmethylcellulose phthalate, methylcellulose, ethylcellulose, shellac, zein, components derived from yeast cellular walls, water soluble polysaccharides, fats, oils, waxes, and chitosan.

29. A process for producing a coated drug, comprising a step for coating a drug with a coating liquid containing from 1 to 50% by weight of an aqueous shellac coating agent according to any one of claim 1 through claim 12, wherein a shellac solid fraction content within a produced coated drug is within a range from 0.1 to 50% by weight.

30. A glazing composition for oil-based confectionary, which is in a liquid form and comprises an aqueous shellac solution (A) comprising an aqueous shellac coating agent containing a mixture of shellac, a basic amino acid and/or a basic phosphate, a thickener (B), and/or a sugar (C).

31. A glazing composition for oil-based confectionary according to claim 30, wherein said basic amino acid contained within said aqueous shellac solution (A) is one or more materials selected from a group consisting of arginine, lysine, and ornithine.

32. A glazing composition for oil-based confectionary according to claim 30, wherein said basic phosphate contained within said aqueous shellac solution (A) is one or more materials selected from a group consisting of trisodium phosphate, tripotassium phosphate, disodium hydrogenphosphate, dipotassium hydrogenphosphate, tetrasodium pyrophosphate, and tetrapotassium pyrophosphate.

33. A glazing composition for oil-based confectionary according to claim 30, wherein a quantity of said basic amino acid contained within said aqueous shellac solution (A) is within a range from 0.05 to 0.40 parts by weight per 1 part by weight of said shellac.

34. A glazing composition for oil-based confectionary according to claim 30, wherein a quantity of said basic phosphate contained within said aqueous shellac solution (A) is within a range from 0.04 to 0.60 parts by weight per 1 part by weight of said shellac.

35. A glazing composition for oil-based confectionary according to claim 30, wherein said aqueous shellac coating agent accounts for 1 to 40% by weight of said aqueous shellac solution (A).

36. A glazing composition for oil-based confectionary according to claim3 0, wherein said thickener (B) is either one, or a mixture of two or more materials selected from a group consisting of pullulan, xanthan gum, guar gum, locust bean gum, tamarind gum, pectin, carrageenan, tragacanth gum, gum arabic, gelatin, and collagen.

37. A glazing composition for oil-based confectionary according to claim 30, wherein said sugar (C) is either one, or a mixture of two or more materials selected from a group consisting of monosaccharides, disaccharides, oligosaccharides, acid-saccharified starch syrup, enzyme-saccharified starch syrup, and starch decomposition products.

38. A glazing composition for oil-based confectionary according to claim 30, wherein a sugar concentration is within a range from 8 to 80% by weight.

39. A glazing composition for oil-based confectionary according to claim 30, comprising a sugar alcohol instead of said sugar (C).

40. A glazing composition for oil-based confectionary according to claim 39, wherein said sugar alcohol is one, or a mixture of two or more materials selected from a group consisting of reduced starch syrup, sorbitol, maltitol, and xylitol.

41. A glazing composition for oil-based confectionary according to claim 30, which contains essentially no organic solvents.

42. A process for glazing oil-based confectionary, wherein a glazing composition according to claim 30 is applied to oil-based confectionary to be glazed to generate a glaze.

43. A process for glazing oil-based confectionary according to claim 42, comprising the steps of applying a glazing composition to said oil-based confectionary, and polishing.

44. A process for glazing oil-based confectionary according to claim 42, wherein a glazing composition is added and applied while said oil-based confectionary is rolled within a rotary pan, and said glazed oil-based confectionary is subsequently subjected to forced-air drying.

45. A process for glazing oil-based confectionary according to claim 42, wherein said oil-based confectionary is one or more types of granular confectionary selected from a group consisting of chocolate, white chocolate and nut cream.

46. A process for glazing oil-based confectionary according to claim 42, which uses essentially no organic solvents.

47. Glazed oil-based confectionary obtainable using a process for glazing oil-based confectionary according to any one of claim 42 through claim 46.

48. Glazed oil-based confectionary according to claim 47, wherein said oil-based confectionary is one or more types of granular oil-based confectionary selected from a group consisting of chocolate, white chocolate and nut cream.

49. Glazed oil-based confectionary according to claim 47, wherein said oil-based confectionary is granular oil-based confectionary produced by coating edible granules of a material selected from a group consisting of chocolate, oil-based cream, nuts, and candy with a material selected from a group consisting of oil-based cream, chocolate and white chocolate, and performing subsequent molding.
